# EUROPEAN PATENT APPLICATION

(11) **EP 4 471 162 A1**
(43) Date of publication of application: **04.12.2024**
(21) Application number: 23747284.0
(22) Date of filing: 20.01.2023
(51) Int. Cl.: C12Q 1/70, C12Q 1/686

(54) **METHOD FOR DETECTING TARGET NUCLEIC ACIDS IN SAMPLE**

(30) Priority: 26.01.2022 KR 20220011832
(71) Applicant: Seegene, Inc., Seoul 05548 (KR)
(72) Inventor: KWON, Suk-Tae, Seoul 05548 (KR); CHO, Sung Suk, Seoul 05548 (KR); KIM, Sunghui, Seoul 05548 (KR); HAN, Jeongeun, Seoul 05548 (KR)
(74) Representative: Dehns
(86) International application number: PCT/KR2023/001072
(87) International publication number: WO 2023/146243

(57) **Abstract**

The present disclosure relates to a method for detecting target nucleic acids in a sample. The method according to the present disclosure can rapidly detect target nucleic acids and has simplified protocols, and thus can be effectively applied to large hospitals, entrusted testing institutions, research laboratories, and the like at which molecular diagnosis for a large number of samples is required.

## Description

### Technical Field

The present disclosure relates to a method for detecting a target nucleic acid in a sample

### Background Art

Molecular diagnostics is a rapidly growing field in the *in vitro* diagnostic market for early diagnosis of disease. Among them, nucleic acid-based methods have been usefully used for diagnosing the causative genetic factors caused by viral and bacterial infections, *etc.* based on high specificity and sensitivity.

Most nucleic acid-based diagnostic methods involve the amplification of a target nucleic acid (e.g., viral or bacterial nucleic acid). As a representative example, polymerase chain reaction (PCR), which is a method for nucleic acid amplification, comprises repeated cycles of denaturation of double-stranded DNA, annealing of oligonucleotide primers to DNA templates, and extension of the primers with a DNA polymerase (Mullis et al., U.S. Patent Nos. 4,683,195, 4,683,202, and 4,800,159; Saiki et al., (1985) Science 230, 1350-1354).

As other methods for nucleic acid amplification, various methods, such as ligase chain reaction (LCR), strand displacement amplification (SDA), nucleic acid sequence-based amplification (NASBA), transcription mediated amplification (TMA), and rolling-circle amplification (RCA) have been suggested.

Recently, with the global pandemic of severe acute respiratory syndrome coronavirus 2 (SARS-CoV-2), real-time PCR diagnostic kits for the detection of SARS-CoV-2 have been receiving much attention, and there has been an emerging need for more rapid and accurate RNA virus detection methods.

Accordingly, the present inventors have developed a method that can more quickly and accurately detect a target nucleic acid in a sample.

Numerous references and patent documents are referenced and cited throughout this specification. The disclosures of the cited documents and patent documents are incorporated herein by reference in their entirety to more clearly explain the content of the present disclosure and the level of technical field to which the present disclosure pertains.

### Disclosure of Invention

### Technical Problem

The present inventors have attempted to develop a method that can more quickly and accurately detect a target nucleic acid in a sample. As a result, the present inventors have confirmed that it is possible to accurately detect the target nucleic acid in a sample within a short time by way of using a thermostable Sso7d fusion DNA polymerase and an antibody, which is specifically bound to the thermostable Sso7d fusion DNA polymerase so as to inhibit the activity of the thermostable Sso7d fusion DNA polymerase.

Accordingly, an object of the present disclosure is to provide a method for detecting a target nucleic acid in a sample.

Another object of the present disclosure is to provide a composition for detecting a target nucleic acid in a sample.

### Solution to Problem

According to one aspect of the present disclosure, provided is a method for detecting a target nucleic acid in a sample, comprising the following steps:
(a) preparing a reaction mixture by mixing the sample with a composition for detecting the target nucleic acid;
   wherein the composition comprises: (i) an oligonucleotide capable of hybridizing with the target nucleic acid, (ii) a thermostable Sso7d fusion DNA polymerase, and (iii) an antibody specifically bound to the thermostable Sso7d fusion DNA polymerase for inhibiting its activity,
(b) incubating the reaction mixture at 65°C or higher to induce denaturation of the antibody;
   wherein the denaturation of the antibody restores the activity of the thermostable Sso7d fusion DNA polymerase; and
(c) amplifying the target nucleic acid in the reaction mixture.

According to one embodiment of the present disclosure, the sample is a swab sample, a spit sample, or a combination thereof.

According to one embodiment of the present disclosure, the swab sample is a nasopharyngeal swab sample, a nasal swab sample, an oropharyngeal swab sample, a saliva swab sample, or a combination thereof.

According to one embodiment of the present disclosure, the sample is subjected to a crude extraction process prior to the step (a).

According to one embodiment of the present disclosure, the crude extract process comprises incubating the sample at 95°C to 100°C for 1 to 25 minutes.

According to one embodiment of the present disclosure, the thermostable Sso7d fusion DNA polymerase comprises an epitope domain for the antibody.

According to one embodiment of the present disclosure, the target nucleic acid is RNA.

According to one embodiment of the present disclosure, the method further comprises step (a-1) of incubating the reaction mixture at 45°C to 55°C before the step (b) to reverse transcribe the target nucleic acid.

According to one embodiment of the present disclosure, the composition further comprises a reverse transcriptase.

According to one embodiment of the present disclosure, the composition further comprises dNTPs and a buffer.

According to one embodiment of the present disclosure, the buffer comprises a Tris buffer in the pH range of 7.0 to 8.0.

According to one embodiment of the present disclosure, the Tris buffer is comprised in the reaction mixture at a concentration of 60 mM to 120 mM.

According to one embodiment of the present disclosure, the composition further comprises MgCl₂.

According to one embodiment of the present disclosure, the MgCl₂ is comprised in the reaction mixture at a concentration of 6 mM to 9 mM.

According to one embodiment of the present disclosure, the MgCl₂ is separated from the thermostable Sso7d fusion DNA polymerase prior to the step (a).

According to one embodiment of the present disclosure, the composition further comprises MgCl₂, and MgCl₂ is separated from the reverse transcriptase prior to the step (a).

According to one embodiment of the present disclosure, the composition further comprises uracil DNA glycosylase (UDG).

According to one embodiment of the present disclosure, the composition further comprises an RNase inhibitor.

According to one embodiment of the present disclosure, the composition further comprises a stabilizer.

According to one embodiment of the present disclosure, the step (a-1) is performed for 5 to 10 minutes.

According to one embodiment of the present disclosure, the step (b) is performed for 1 to 3 minutes.

According to one embodiment of the present disclosure, the step (c) is performed for 6 to 47 minutes.

According to one embodiment of the present disclosure, the step (c) is performed for 40 to 45 cycles of the process of 2 to 5 seconds at 95°C and 5 to 20 seconds at 60°C.

According to one embodiment of the present disclosure, the steps (a-1), (b), and (c) are performed for a total of 12 to 60 minutes.

According to one embodiment of the present disclosure, the method detects a plurality of target nucleic acids.

According to another aspect of the present disclosure, provided is a composition, comprising:
(a) an oligonucleotide capable of hybridizing with a target nucleic acid;
(b) a thermostable Sso7d fusion DNA polymerase; and
(c) an antibody specifically bound to the thermostable Sso7d fusion DNA polymerase for inhibiting activity of the thermostable Sso7d fusion DNA polymerase.

According to one embodiment of the present disclosure, the antibody is denatured at 65°C or higher.

According to one embodiment of the present disclosure, the denaturation of the antibody restores the activity of the thermostable Sso7d fusion DNA polymerase.

### Advantageous Effects of Invention

The features and advantages of the present disclosure are summarized as follows:
(a) The method of the present disclosure enables faster and more accurate detection of a target nucleic acid in a sample by using a thermostable Sso7d fusion DNA polymerase and an antibody, which is specifically bound to the thermostable Sso7d fusion for inhibiting the activity of thermostable Sso7d fusion DNA polymerase.
(b) The method of the present disclosure, by using a crude extraction sample without nucleic acid purification, not only enables faster detection of a target nucleic acid, but also has a simplified protocol, and thus, it can be usefully applied in large hospitals, contract testing organizations, research institutes, *etc.* where molecular diagnosis of large quantities of samples is required.
(c) The method of the present disclosure can significantly reduce the detection time through optimization, such as use of high-concentration Tris buffer, use of an RNase inhibitor, or separate storage of MgCl₂ and the enzyme.

### Best Mode for Carrying out the Invention

An aspect of the present disclosure is to provide a method for detecting a target nucleic acid in a sample comprising the following steps:
(a) preparing a reaction mixture by mixing the sample with a composition for detecting the target nucleic acid,
   wherein the composition comprises: (i) an oligonucleotide capable of hybridizing with the target nucleic acid, (ii) a thermostable Sso7d fusion DNA polymerase, and (iii) an antibody specifically bound to the thermostable Sso7d fusion DNA polymerase for inhibiting its activity;
(b) incubating the reaction mixture at 65°C or higher to induce denaturation of the antibody,
   wherein the denaturation of the antibody restores the activity of the thermostable Sso7d fusion DNA polymerase; and
(c) amplifying the target nucleic acid in the reaction mixture.

Hereinafter, the present disclosure will be described in more detail according to each step.

### Step (a): Preparation of reaction mixture

In the step (a) of the present disclosure, a reaction mixture is prepared by mixing a sample with a composition for detecting a target nucleic acid.

As used herein, the term "sample" may refer to any analyte which comprises or is suspected of comprising the nucleic acid to be detected. For example, the sample may comprise biological samples (*e.g*., cells, tissues, and body fluids) and non-biological samples (*e.g*., foods, water, and soil). The biological samples may include, for example, viruses, bacteria, tissues, cells, blood (including whole blood, plasma, and serum), lymph, bone marrow fluid, saliva, sputum, swab, aspiration, milk, urine, stool, ocular humor, semen, brain extracts, spinal fluid, joint fluid, thymus fluid, bronchoalveolar lavage fluid, ascites, and amniotic fluid, but is not limited thereto.

According to one embodiment, the sample may be obtained from a subject, particularly a mammal, and more particularly a human, and it may be, for example, a swab, saliva, sputum, aspiration, bronchoalveolar lavage (BAL), gargle, or blood, but is not limited thereto.

As used herein, the term "subject" refers to an entity suspected of including a target nucleic acid (*e.g*., a specific pathogen) to be detected using the method of the present disclosure. Examples of the subject may comprise without limitation, mammals (*e.g*., dogs, cats, rodents, primates and humans), and particularly, the subject may be humans.

As used herein, the term sample may be used interchangeably with "specimen" herein.

In one embodiment, the sample derived from the subject is a swab sample, a spit sample, or a combination thereof.

In one embodiment, the swab sample is a nasopharyngeal swab sample, a nasal swab sample, an oropharyngeal swab sample, a saliva swab sample, or a combination thereof.

In one embodiment, the spit sample is a saliva sample or sputum sample.

As used herein, the term "nucleic acid", "nucleic acid sequence", or "nucleic acid molecule" refers to a deoxyribonucleotide or ribonucleotide polymer in single-stranded or double-stranded form, and the nucleotide may comprise a derivative of a natural nucleotide, a non-natural nucleotide, or a modified nucleotide that can function in the same manner as in a naturally occurring nucleotide.

As used herein, the term "target nucleic acid", "target nucleic acid sequence", or "target sequence" refers to a nucleic acid sequence to be detected. The target nucleic acid sequence may hybridize with the oligonucleotides comprised in the composition for detecting a target nucleic acid used in the method of the present disclosure.

According to one embodiment, the target nucleic acid may be a nucleic acid of a human, an animal, a plant, or a microorganism. The microorganism may be a fungus, a protozoan, a bacterium, a virus, an alga, *etc.*

According to one embodiment, the target nucleic acid may be a nucleic acid of a virus, and specifically, the target nucleic acid may be a nucleic acid of an RNA virus.

According to one embodiment, the target nucleic acid may be a nucleic acid of a respiratory virus. For example, the target nucleic acid may comprise a nucleic acid of influenza virus, a nucleic acid of a respiratory syncytial virus (RSV), a nucleic acid of an adenovirus, a nucleic acid of an enterovirus, a nucleic acid of a parainfluenza virus, a nucleic acid of a metapneumovirus (MPV), a nucleic acid of a bocavirus, a nucleic acid of a rhinovirus, and/or a nucleic acid of a coronavirus, *etc.*

According to one embodiment, the target nucleic acid may be a nucleic acid of severe acute respiratory syndrome coronavirus 2 (SARS-CoV-2).

According to one embodiment, the nucleic acid of SARS-CoV-2 may be selected from the group consisting of the E gene, N gene, RdRP gene, S gene, and a combination thereof.

According to one specific embodiment, the target nucleic acid may be the E gene, the RdRP gene, and the N gene of SARS-CoV-2.

According to one embodiment, the sample may be subjected to nucleic acid extraction and/or purification processes known in the art for an efficient amplification reaction (see: Sambrook, J. et al., Molecular Cloning. A Laboratory Manual, 3rd ed. Cold Spring Harbor Press (2001)). The nucleic acid extraction and/or purification process may vary depending on the type of sample.

According to another embodiment, the sample may be analyzed directly without extracting or purifying the nucleic acid in the sample. For example, the method disclosed in Pannacio et al. (Nucleic Acids Res. 21(19): 4656, 1993) and Pandori et al. (BMC Infect Dis. 6: 104, 2006) may be used.

In one embodiment, the sample may be subjected to a crude extraction process prior to the step (a). For example, the sample is a crude extraction sample that has undergone a crude extraction process without purification of nucleic acids.

In one embodiment, as the crude extraction process, various conventional methods disclosed may be used, for example, the method disclosed in WO2021/261864. WO2021/261864 discloses a method for providing a preparation (*i.e*., a reaction mixture) for detecting a target nucleic acid sequence, in which a sample (*e.g*., a specimen stored in a transport medium) is incubated at 95°C to 100°C for 1 to 25 minutes to lyse the sample, and the lysate is mixed with a composition for detecting the target nucleic acid.

In one embodiment, the crude extraction process comprises incubating the sample at 95°C to 100°C for 1 to 25 minutes.

According to one embodiment, the crude extraction process further comprises treating the sample with proteinase K. Specifically, the crude extraction process comprises treating the sample with proteinase K to lower the viscosity of the sample (*e.g*., a specimen), followed by incubation at 95°C to 100°C for 1 to 25 minutes.

According to one embodiment, the crude extraction process further comprises the step of incubating at 95°C to 100°C for 1 to 25 minutes followed by cooling to 2°C to 10°C . Subsequently, the cooled crude extraction sample is mixed with the composition for detecting a target nucleic acid.

According to one embodiment, the crude extraction sample may be mixed with the composition for detecting a target nucleic acid at a volume ratio of 1:1 to 1:20, specifically 1:2 to 1:10, and more specifically at a volume ratio of 1:2 to 1:4.

According to one embodiment, the crude extraction sample may be mixed with the composition for detecting a target nucleic acid without dilution.

According to one embodiment, the crude extraction sample may be diluted with water or a buffer and mixed with the composition for detecting a target nucleic acid. The dilution -relieves inhibition of a reaction inhibitor(s) that may be present in the crude extraction sample (*e.g*., a reaction inhibitor(s) that may be present in the transport medium) during the reaction by mixing with the composition for detecting a target nucleic acid.

The water used for dilution is, for example, purified water, nuclease-free-water (*e.g*., RNase-free water), or distilled water.

The buffer used for dilution is, for example, TE buffer.

According to one embodiment, the crude extraction sample may be diluted 1- to 10-fold. Specifically, the crude extraction sample may be diluted 2- to 8- fold, 2- to 7- fold, 2- to 6-fold, 2- to 5-fold, or 2- to 4-fold, and more specifically, may be diluted 2- to 4-fold. For example, when the crude extraction sample is diluted 2-fold, the crude extraction sample may be diluted 2-fold by mixing the crude extraction sample and water (or buffer) in a 1:1 ratio.

As used herein, the term "a composition for detecting a target nucleic acid" refers to a substance, which comprises a combination of components that are used to detect a target nucleic acid (*e.g*., a nucleic acid of an RNA virus) present in a sample (*e.g*., a sample from an individual infected with the RNA virus). The composition for detecting a target nucleic acid may comprise components for amplifying the target nucleic acid and components that generate a signal from the amplified target nucleic acid. In particular, when the target nucleic acid is a nucleic acid of an RNA virus, the composition for detecting a target nucleic acid in the RNA virus may comprise components for reverse transcribing the target RNA into target DNA, components for amplifying the reverse-transcribed target DNA, and components for generating a signal from the amplified target DNA. The optimal amounts of the components to be used in a particular reaction may readily be determined by those skilled in the art who are well aware of the advantages of the present disclosure. Additionally, the components of the composition for detecting a target nucleic acid may be present in individual containers or a plurality of components may be present in one container.

The composition for detecting a target nucleic acid comprises (i) an oligonucleotide capable of hybridizing with the target nucleic acid, (ii) a thermostable Sso7d fusion DNA polymerase, and (iii) an antibody specifically bound to the thermostable Sso7d fusion DNA polymerase for inhibiting its activity. Hereinafter, the components comprised in the composition for detecting a target nucleic acid according to the present disclosure will be described in detail.

### (a) Oligonucleotide capable of hybridizing with target nucleic acid

In one embodiment, the oligonucleotide capable of hybridizing to a target nucleic acid comprises oligonucleotides (*e.g*., primers or probes) for amplifying and/or detecting a target nucleic acid.

In a certain embodiment, the oligonucleotide capable of hybridizing to a target nucleic acid is a primer, a probe, or a combination thereof.

As used herein, the term "primer" refers to an oligonucleotide that can serve as a starting point for synthesis under the conditions where the synthesis of a primer extension product complementary to a target nucleic acid sequence (template) is induced, that is, under the conditions with the presence of a polymerizing agent (*e.g.*, nucleotides and DNA polymerase) and of appropriate temperature and pH. Primers should be sufficiently long to prime the synthesis of the extension product in the presence of a polymerizing agent. The appropriate length of the primer depends on a number of factors, such as a temperature, a field of application, and a source of the primer.

In one embodiment, the oligonucleotide may have a length of 5 bp to 1,000 bp, 5 bp to 900 bp, 5 bp to 800 bp, 5 bp to 700 bp, 5 bp to 600 bp, 5 bp to 500 bp, 5 bp to 400 bp, 5 bp to 300 bp, 5 bp to 200 bp, 5 bp to 150 bp, 5 bp to 100 bp, 5 bp to 90 bp, 5 bp to 80 bp, 5 bp to 70 bp, 5 bp to 60 bp, 5 bp to 50 bp, 5 bp to 40 bp, 5 bp to 30 bp, 5 bp to 20 bp, 5 bp to 10 bp, 10 bp to 1,000 bp, 10 bp to 900 bp, 10 bp to 800 bp, 10 bp to 700 bp, 10 bp to 600 bp, 10 bp to 500 bp, 10 bp to 400 bp, 10 bp to 300 bp, 10 bp to 200 bp, 10 bp to 150 bp, 10 bp to 100 bp, 10 bp to 90 bp, 10 bp to 80 bp, 10 bp to 70 bp, 10 bp to 60 bp, 10 bp to 50 bp, 10 bp to 40 bp, 10 bp to 30 bp, 10 bp to 20 bp, 15 bp to 1,000 bp, 15 bp to 900 bp, 15 bp to 800 bp, 15 bp to 700 bp, 15 bp to 600 bp, 15 bp to 500 bp, 15 bp to 400 bp, 15 bp to 300 bp, 15 bp to 200 bp, 15 bp to 150 bp, 15 bp to 100 bp, 15 bp to 90 bp, 15 bp to 80 bp, 15 bp to 70 bp, 15 bp to 60 bp, 15 bp to 50 bp, 15 bp to 40 bp, or 15 bp to 30 bp, but is not limited thereto.

As used herein, the term "probe" refers to a single-stranded nucleic acid molecule which comprises a portion or portions substantially complementary to a target nucleic acid sequence. According to one embodiment, the 3'-end of a probe is "blocked" to prevent its extension. The blocking may be achieved according to conventional methods. For example, the blocking may be accomplished by adding a chemical moiety (*e.g.*, biotin, a label, a phosphate group, an alkyl group, a non-nucleotide linker, a phosphorothioate, or an alkane-diol residue) to the 3'-hydroxyl group of the last nucleotide. Alternatively, the blocking may be performed by removing the 3'-hydroxyl group of the last nucleotide or using a nucleotide without a 3'-hydroxyl group (*e.g*., a dideoxynucleotide).

The primer or probe may be single stranded. The primer or probe comprises a deoxyribonucleotide, a ribonucleotide, or a combination thereof. The primer or probe used in the present disclosure may comprise naturally-occurring dNMP (*i.e*., dAMP, dGMP, dCMP, and dTMP), a modified nucleotide, or an unnatural nucleotide. A representative example of a base for such a non-natural nucleotide is inosine, which is known as a universal base.

The primer or probe does not need to have a perfectly complementary sequence to that of the template, but it is sufficient to have complementarity within the range that can hybridize with the template and perform the original function of the primer or probe.

The term "annealing" or "priming" refers to the apposition of an oligonucleotide or nucleic acid to a template nucleic acid, and the apposition causes a polymerase to polymerize the nucleotides to form a nucleic acid molecule complementary to the template nucleic acid or a portion thereof.

As used herein, the term "hybridization" refers to the formation of two single-stranded polynucleotides into a double-strand through non-covalent bonds between complementary nucleotide sequences under predetermined hybridization conditions.

In one embodiment, the oligonucleotide capable of hybridizing to the target nucleic acid comprises a hybridizing nucleotide sequence with the target nucleic acid sequence.

As used herein, the term "a hybridizing nucleotide sequence with a target nucleic acid sequence" refers to "a nucleotide sequence that hybridizes with a target nucleic acid sequence".

According to one embodiment, the hybridizing nucleotide sequence of the oligonucleotide comprises a sequence that hybridizes with the target nucleic acid sequence under given hybridization conditions.

According to one embodiment, the oligonucleotide comprises a hybridizing nucleotide sequence that hybridizes with a target nucleic acid sequence under stringent conditions.

In the present disclosure, appropriate hybridization conditions or stringent conditions may be determined through a series of optimization procedures. These procedures are performed by way of a series of processes by those skilled in the art to establish a protocol for use in a laboratory. For example, conditions such as a temperature, a concentration of components, time required for hybridization and washing, buffer components and pH and ionic strength thereof rely on various factors, such as the length and GC amount of the oligonucleotide and the target nucleic acid sequence. The specific conditions for hybridization may be found in Joseph Sambrook, et al., Molecular Cloning, A Laboratory Manual, Cold Spring Harbor Laboratory Press, Cold Spring Harbor, N.Y. (2001); and M.L.M. Anderson, Nucleic Acid Hybridization, Springer-Verlag New York Inc. N.Y. (1999).

In one embodiment, the stringent conditions comprise temperature conditions including the temperatures selected within a certain range based on the sequence Tm value of the region within the target nucleic acid sequence to be hybridized with the oligonucleotide, for example, within the Tm values ±1°C, ±2°C, ±3°C, ±4°C, ±5°C, and ±7°C.

In one embodiment, the stringent conditions may include the following conditions:
(1) 5× SSC, 5X Denhardt's solution, 0.5% SDS, 50% formamide 32°C, (2) 5× SSC, 5× Denhardt's solution, 0.5% SDS, 50% formamide, 42°C, (3) 5× SSC, 1% SDS, 50 mM Tris-HCl (pH 7.5), 50% formamide 42°C, (4) 5× SSC, 5× Denhardt's solution, 0.5% SDS, 50% formamide 50°C, (5) 0.2× SSC, 0.1% SDS 60°C, (6) 0.2× SSC, 0.1% SDS 62°C, (7) 0.2× SSC, 0.1% SDS 65°C, or (8) 0.1× SSC, 0.1% SDS 65°C, but the stringent conditions are not limited thereto.

According to one embodiment, the hybridizing nucleotide sequence of the oligonucleotide may be determined depending on the hybridization conditions used and the nucleic acid sequence to be hybridized with the oligonucleotide (*i*.*e.*, the target nucleic acid sequence).

According to one embodiment, the stringent condition is the temperature at which the entire oligonucleotide hybridizes with the target nucleic acid sequence but does not hybridize with a non-target nucleic acid sequence.

According to one embodiment, the stringent condition is the temperature at which a portion of the oligonucleotide (*e.g*., the 5' end or the 3' end) hybridizes with the target nucleic acid sequence but does not hybridize with a non-target nucleic acid sequence.

In this specification, the expression that one oligonucleotide "comprises a hybridizing nucleotide sequence" with respect to another oligonucleotide means that all or part of the one oligonucleotide has the complementary nucleotide sequence required for the hybridization with all or part of the another oligonucleotide.

According to one embodiment, the oligonucleotide may comprise a hybridizing nucleotide sequence that is complementary to the target nucleic acid sequence.

The term "complementary" means that a primer or probe is sufficiently complementary to selectively hybridize with a target nucleic acid sequence under predetermined annealing or stringency conditions, and it encompasses both substantially complementary and perfectly complementary, and specifically means perfectly complementary.

The term "substantially complementary" may comprise 1 to 4 mismatches, 1 to 3 mismatches, or 1 to 2 mismatches.

When referring to hybridization between a portion within one oligonucleotide and another oligonucleotide, the portion of one oligonucleotide may be regarded as an individual oligonucleotide and the hybridization between different oligonucleotides may be expressed.

Hybridization occurs when the complementarity at a portion where hybridization occurs (a portion where a double-strand is formed) between two nucleic acid sequences is perfect (a perfect match), or hybridization may occur even if mismatch bases are present (e.g., 1 to 4 mismatches, 1 to 3 mismatches, or 1 to 2 mismatches). The degree of complementarity required for hybridization may vary depending on hybridization reaction conditions, and particularly, is controlled by temperature.

For example, the degree of complementarity required for hybridization may be at 70% or more, 75% or more, 80% or more, 85% or more, 90% or more, or 95% or more.

According to one embodiment, the degree of complementarity or the number of mismatches between two oligonucleotides is determined based on the portion being hybridized.

As used herein, there is no difference between the terms "hybridization" and "annealing", and in this specification, these terms are used interchangeably.

The oligonucleotides used in the present disclosure may have various structures known in the art; for example, they may have a hairpin structure or stem-loop structure, may have a linker (spacer), or may comprise a tag that does not hybridize with a target nucleic acid.

### (b) Thermostable Sso7d fusion DNA polymerase

The composition for detecting a target nucleic acid according to the present disclosure comprises a thermostable DNA polymerase. The thermostable DNA polymerase comprises thermostable DNA polymerases that can be obtained from a variety of bacterial species, for example, DNA polymerases from *Thermus aquaticus* (Taq), *Thermus thermophiles* (Tth), *Thermus filiformis, Thermis flavus, Thermococcus literalis,* or *Pyrococcus furiosus* (Pfu). Most of the DNA polymerases described above are commercially available.

The thermostable DNA polymerase may be wild-type or a mutant modified to improve its activity, *etc.* In one embodiment, the thermostable DNA polymerase is wild-type Taq DNA polymerase.

In one embodiment, the thermostable DNA polymerase is a DNA polymerase with improved processivity.

The processivity of a polymerase refers to the ability of the enzyme to continuously synthesize new nucleic acids by binding to the substrate (*i.e.*, DNA or RNA) used as a template without being detached therefrom during nucleic acid replication. This processivity is also called persistence.

In one embodiment, the DNA polymerase with improved processivity comprises the polymerase described in International Patent Publication No. WO2001/092501. These polymerases with improved processivity exhibit improved processivity due to the presence of a sequence-non-specific double stranded DNA binding domain bound to the polymerase or the enzymatic domain of the polymerase.

In one embodiment, the sequence-non-specific double stranded DNA binding domain may be Sso7d or Sac7d protein of about 7,000 kD MW derived from thermotolerant organisms, for example, hyperthermophilic archaea *Sulfolobus solfataricus* and *S*. *acidocaldarius* (see WO 2001/092501).

In one embodiment, the composition for detecting a target nucleic acid comprises a thermostable Sso7d fusion DNA polymerase.

In the thermostable Sso7d fusion DNA polymerase described above, the Sso7d may be fused in various ways as long as it does not affect the activity or function of the DNA polymerase. In one embodiment, the Sso7d is linked to the N-terminus of the amino acid sequence of DNA polymerase. In another embodiment, the Sso7d is linked to the C-terminus of the amino acid sequence of DNA polymerase. In still another embodiment, the Sso7d is linked to the inside of the amino acid sequence of DNA polymerase.

### (c) Antibody specifically bound to the thermostable Sso7d fusion DNA polymerase

The composition for detecting a target nucleic acid according to the present disclosure comprises an antibody, which is specifically bound to the thermostable Sso7d fusion DNA polymerase, to inhibit activity of the thermostable Sso7d fusion DNA polymerase.

Recently, the "hot-start" approach has been widely used with thermostable polymerases to improve the accuracy of amplification methods using polymerases (*e.g*., PCR). For example, there is a method using chemically modified DNA polymerase, and this technology was developed by Roche (Birch D.E. et al., 1996, Nature 381: 445-446, Birch D.E. et al., 1997, U.S. Patent USP 5677152) and Qiagen (Ivanov Igor et al., 2001, U.S. Patent USP 6183998), respectively. According to this method, the activity of Taq DNA polymerase is inhibited by chemical modification, and then the activity of the enzyme is reactivated through the initial reactivation process of a PCR reaction (*e.g*., 10 minutes at 95°C) to proceed with PCR. However, only about 30% of the enzyme is reactivated at the beginning of the PCR reaction, and the reactivation by high temperature causes depurination of the template DNA, which has a disadvantage in that it makes the amplification of a long length impossible.

In another hot-start method, U.S. Patent No. 5,338,671 describes the use of antibodies specific for thermostable DNA polymerase to inhibit DNA polymerase activity at low temperatures (*e.g*., below 70°C). Specifically, the antibody reacts with DNA polymerase at room temperature and inhibits the activity of DNA polymerase. As the temperature gradually increases, the antibody is denatured and detached from the enzyme, and the activity of the polymerase is reactivated above a certain temperature. That is, when the temperature exceeds a certain temperature, the antibody is detached from the polymerase, and the activity of the polymerase is restored, and an oligonucleotide (*e.g*., a primer) capable of hybridizing with a target nucleic acid may accurately hybridize with the target nucleic acid, thereby allowing only the target nucleic acid to be specifically amplified. The hot-start method using an antibody has an advantage in that the reaction time for reactivation of the initial enzyme is short and the enzyme reactivation rate is also high compared to the hot-start method using chemical modification.

In order to improve the accuracy of the amplification reaction, the present inventors have adopted, among various applicable hot-start methods, a hot-start method using an antibody that can reactivate the polymerase within a short time. Through this, it is possible to rapidly detect a target nucleic acid with higher accuracy.

In the thermostable Sso7d fusion DNA polymerase according to the present disclosure, an antibody specific to the thermostable DNA polymerase is bound thereto.

In one embodiment, the thermostable Sso7d fusion DNA polymerase comprises an epitope domain for the antibody.

In one embodiment, the antibody specifically binds to the thermostable Sso7d fusion DNA polymerase and inhibits its activity. As used herein, the term "antibody" includes polyclonal antibodies, monoclonal antibodies, and antigenic compound-binding fragments of these antibodies, and for example, comprises Fab, F(ab')2, Fd, Fv, scFv, bispecific antibodies, and antibody minimal recognition units, and single-stranded derivatives of these antibodies and fragments thereof. The term "antibody" comprises natural and non-natural antibodies, and for example, comprises chimeric, bifunctional antibodies, and humanized antibodies, and related synthetic isoforms.

Examples of the above antibodies may comprise various commercially available antibodies, for example, hot-start Taq monoclonal antibody and platinum Taq monoclonal antibody (Thermo Fisher), and JumpStart^{™} Taq antibody (Sigma-Aldrich), but are not limited thereto.

### (d) Reverse transcriptase (RTase)

In one embodiment, the composition for detecting a target nucleic acid further comprises a reverse transcriptase (RTase).

As used herein, the term "reverse transcriptase", which is also called RNA-dependent DNA polymerase, refers to an enzyme that uses RNA as a template and synthesizes complementary DNA.

In order to detect RNA viruses, a reverse transcription reaction to generate cDNA from an RNA template is essential. The specific details are disclosed in Joseph Sambrook, et al., Molecular Cloning, A Laboratory Manual, Cold Spring Harbor Laboratory Press, Cold Spring Harbor, N.Y. (2001); and Noonan, K. F. et al., Nucleic Acids Res. 16:10366 (1988).

In one embodiment, as the reverse transcriptase, reverse transcriptases originating from a variety of sources may be used, for example, avian myeloblastosis virus-derived reverse transcriptase (AMV RTase), murine leukemia virus-derived reverse transcriptase (MuLV RTase) and Rous-associated virus 2 reverse transcriptase (RAV-2 RTase) may be used, but the reverse transcriptase is not limited thereto.

A reverse transcriptase requires a primer to synthesize cDNA from an RNA template. There are three main types of primers used in reverse transcription reactions: (i) an oligo dT primer that is annealed to the poly A tail of mRNA and synthesizes cDNA from the 3'-end, (ii) a random primer made of random nucleotides of 6 nt to 9 nt in size to initiate synthesis in all regions of RNA; and (iii) a target-specific primer that synthesizes only target cDNA.

According to one embodiment, the composition may comprise a primer for a reverse transcription reaction, and the primer for the reverse transcription reaction may be a target-specific primer.

In one embodiment, the target-specific primer for the reverse transcription reaction is used to synthesize cDNA in the reverse transcription reaction, and subsequently, it may be used as a primer pair for the amplification of the obtained cDNA by pairing with another primer (e.g., a forward primer or a reverse primer) in the amplification reaction of the target nucleic acid sequence.

### (e) dNTPs

In one embodiment, the composition for detecting a target nucleic acid further comprises dNTPs. The dNTPs, which refer to nucleoside triphosphates that constitute nucleic acids, consist of dATP, dGTP, dCTP, dUTP, and dTTP. The five types of nucleoside triphosphates may be present in the same amount in the composition for detecting a target nucleic acid.

### (f) Buffer

In one embodiment, the composition for detecting a target nucleic acid further comprises a buffer.

In one embodiment, the buffer comprises Tris buffer of pH 7 to pH 8.

In one embodiment, the composition for detecting a target nucleic acid of the present disclosure comprises the buffer in an amount such that the buffer is present at a relatively high concentration of 60 mM to 120 mM in the reaction mixture. A typical composition for detecting a target nucleic acid (*e.g.*, a PCR composition) includes a buffer in an amount such that the buffer is present at a concentration of 10 mM to 50 mM in the reaction mixtuee (Kangfu Yu et al., Nucleic Acids Research, Vol. 20, No. 10, 1992; Benjamin D. Cobb et al., Nucleic Acids Research, Vol. 22, No. 18 3801 -3805, 1994; Carl T. Wittwer et al., METHODS 25, 430-442, 2001;). In contrast, the relatively high concentration of Tris buffer used in one embodiment of the present disclosure shows the effect of improving the detection specificity and signal intensity of the target nucleic acid.

In one embodiment, the Tris buffer is comprised in the reaction mixture to have a concentration of 60 mM to 120 mM, and specifically, it may be at a concentration of 70 mM to 120 mM, 80 mM to 120 mM, 60 mM to 110 mM, 70 mM to 110 mM, or 80 mM to 110 mM.

### (g) Uracil DNA glycosylase (UDG)

In one embodiment, the composition for detecting a target nucleic acid further comprises UDG.

In the case of a reverse transcription reaction or nucleic acid amplification reaction, carry-over contamination may occur where the previous reaction product(s) (*e.g*., cDNA or an amplicon(s)) contaminate(s) the reaction to be newly performed through various routes. That is, there may be a problem in that the carry-over contaminant may act as a template in the reaction to be newly performed and may thus be reverse-transcribed or amplified thus showing a false positive result as if it were positive even when it is actually negative.

Such carry-over contamination may be prevented by using uracil DNA glycosylase (UDG). Specifically, among dNTPs, dUTP is used instead of dTTP to perform a reverse transcription reaction or amplification reaction. The resulting products (*i.e*., cDNA or amplicons) comprise dUTP, and previous products including dUTP are hydrolyzed by treating with UDG before performing the new reaction. As a result, carry-over contamination can be prevented.

As an example of a suitable UDG, heat labile uracil DNA glycosylase (UDG) may be used. The heat labile UDG may comprise UDG derived from cryophilic organisms, for example, UDG derived from psychrophilic bacteria or Alaskan cod, but is not limited thereto. These enzymes have the characteristic of being quickly and irreversibly inactivated when exposed to a temperature of 50°C or 55°C, respectively. Therefore, the heat labile UDG is inactivated before the reverse transcription reaction begins, so that only carry-over contaminants can be removed without affecting the cDNA generation process by the reverse transcription reaction.

### (h) RNase inhibitor

In one embodiment, the composition for detecting a target nucleic acid further comprises an RNase inhibitor.

Examples of RNase inhibitors may comprise various commercially available RNase inhibitors, for example, may comprise RNase inhibitors (Cat. No. N8080119, ThermoFisher), Solg^{™} RNase Inhibitor (SolGent), Recombinant RNase Inhibitors (TaKaRa), *etc*., but RNase inhibitors are not limited thereto.

### (i) MgCl₂

In one embodiment, the composition further comprises MgCl₂.

Mg²⁺ is known to affect polymerase activity, primer annealing, fidelity of synthesized DNA, formation of primer dimers, *etc.* In particular, Mg²⁺ is a cofactor of polymerase and is required for polymerase activity.

In one embodiment, the MgCl₂ is comprised in the composition to have a concentration of 6 mM to 9 mM in the reaction mixture.

In general, PCR reactions require Mg²⁺ at a concentration of 1 mM to 5 mM (Carl T. Wittwer et al., METHODS 25, 430-442, 2001), and it is known that if Mg²⁺ is added in an excessive amount, it actually inhibits enzyme activity. Additionally, it has been known that an excessive amount of Mg²⁺ stabilizes annealing between primers and non-targets, and produces non-specific amplicons, thereby reducing the specificity of a PCR reaction, and that an excessive amount of Mg²⁺ stabilizes double strands of DNA and prevents complete denaturation of DNA (*i.e*., the denaturation step in PCR), thereby reducing the yield of PCR products (Benjamin D. Cobb et al., Nucleic Acids Research, Vol. 22, No. 18 3801 -3805, 1994; P. Markoulatos et al., Journal of Clinical Laboratory Analysis 16:47-51, 2002;).

However, the present inventors have found that enzyme activity can be maximized within a short time by storing the MgCl₂ in a separate container from thermostable Sso7d fusion DNA polymerase and a reverse transcriptase prior to the step (a); and using the MgCl₂ by mixing with the thermostable Sso7d fusion DNA polymerase and the reverse transcriptase to be comprised in the reaction mixture at a relatively high concentration of 6 mM to 9 mM in the step (a).

In one embodiment, the MgCl₂ is separated from the thermostable SSo7d fusion DNA polymerase prior to the step (a).

In one embodiment, the MgCl₂ is separated from the reverse transcriptase prior to the step (a).

In one embodiment, the MgCl₂ is stored in a separate container from the thermostable Sso7d fusion DNA polymerase and the reverse transcriptase prior to the step (a), and mixed with the thermostable Sso7d fusion DNA polymerase and the reverse transcriptase in the step (a).

### (j) Others

In one embodiment, the composition for detecting a target nucleic acid further comprises a stabilizer. The stabilizer comprises, for example, sucrose and glycerol, but is not limited thereto.

### Step (a-1): Reverse transcription reaction

When the target nucleic acid is RNA, the method further comprises step (a-1), in which the target nucleic acid is reverse-transcribed by incubating the reaction mixture at 45°C to 55°C prior to the step (b).

Step (a-1) is a step of generating cDNA for the RNA target nucleic acid. The reverse transcription reaction of the step (a-1) is performed using the reverse transcriptase and the primers comprised in the composition for detecting a target nucleic acid prepared in the step (a).

In one embodiment, the incubation in the step (a-1) is performed at an activation temperature to allow the reverse transcriptase to function. In one embodiment, the incubation in the step (a-1) is optionally performed at an inactivation temperature to render the UDG to be used non-functional. In one embodiment, the incubation in the step (a-1) is performed at a temperature selected considering both the activation of reverse transcriptase and the inactivation of UDG.

In a certain embodiment, the incubation of the step (a-1) is performed at 47°C to 53°C. In a certain embodiment, the incubation of the step (a-1) is performed at 50°C.

In one embodiment, the step (a-1) may be performed for 5 to 20 minutes, and specifically, the step (a-1) may be performed for 5 to 10 minutes.

### Step (b): Induction of denaturation of antibody

In the step (b), the reaction mixture of the step (a) or the step (a-1) is incubated at 65°C or higher to induce denaturation of the antibody. The denaturation of the antibody restores the activity of the thermostable Sso7d fusion DNA polymerase. The step (b) may be explained with reference to the description of the hot-start method using the antibody in the step (a) described above. Therefore, in order to avoid excessive complexity of the present specification, the description of common contents between them is omitted herein.

In one embodiment, the step (b) is performed for 1 to 3 minutes.

As described above, the hot-start method using an antibody denatures the antibody and restores the activity of the polymerase even within a short time. Therefore, the step (b) may be performed during the denaturation process of the double-stranded nucleic acid in the step (c) described below.

### Step (c): Amplification of target nucleic acid

In the step (c), the target nucleic acid is amplified using the reaction mixture of the step (b).

The amplification of nucleic acids may be performed according to various primer-involving nucleic acid amplification methods known in the art. Specifically, the amplification is performed according to polymerase chain reaction (PCR), and the PCR methods are disclosed in U.S. Patent Nos. 4,683,195, 4,683,202, and 4,800,159. Another example includes ligase chain reaction (LCR)) (U.S. Patent Nos. 4,683,195 and 4,683,202; PCR Protocols: A Guide to Methods and Applications (Innis et al., eds, 1990)), strand displacement amplification (SDA) (Walker, et al. Nucleic Acids Res. 20(7): 1691-6 (1992); Walker PCR Methods Appl 3(1): 1-6 (1993)), transcription-mediated amplification (Phyffer, et al., J. Clin. Microbiol. 34: 834-841 (1996); Vuorinen, et al., J. Clin. Microbiol. 33:1856-1859 (1995)), helicase dependent amplification (HAD) (M. Vincent, Y. Xu and H. Kong, EMBO Rep., 2004, 5, 795-800), nucleic acid sequence-based amplification (NASBA) (Compton, Nature 350(6313):91-2 (1991)), rolling circle amplification (RCA) (Lisby, Mol. Biotechnol. 12(1):75-99 (1999); Hatch et al., Genet. Anal. 15(2): 35-40 (1999)), Q-Beta Replicase (Lizardi et al., BiolTechnology 6:1197 (1988)), loop-mediated isothermal amplification (LAMP) (Y. Mori, H. Kanda and T. Notomi, J. Infect. Chemother., 2013, 19, 404-411), recombinase polymerase amplification (RPA) (J. Li, J. Macdonald and F. von Stetten, Analyst, 2018, 144, 31-67), *etc.*

In one embodiment, the amplification of the target nucleic acid may be performed by a nucleic acid amplification method involving a change in temperature, for example, a PCR method. In this nucleic acid amplification method, a cycle including a denaturing step, a primer annealing step, and an extension (or amplification) step may be repeated several to dozens of times.

In one embodiment, the amplification of a target nucleic acid may be performed by a nucleic acid amplification method that does not involve a change in temperature, that is, an isothermal amplification method.

Throughout the detailed description herein, PCR is mainly described as an example of a target nucleic acid amplification method, but those skilled in the art will recognize that various nucleic acid amplification reactions may be applied in addition to PCR.

In one embodiment, the amplification of a target nucleic acid may be performed by a PCR method, and specifically, by a Fast PCR method.

As used herein, the term "Fast PCR", which is one of the PCR techniques, may refer to any PCR technique that can amplify a gene within a short time by simplifying the repetitive process of the amplification step. The Fast PCR may be achieved by controlling various factors, such as the extension rate of DNA polymerase, the ramp speed of a thermocycler, and the complexity of a template. For example, instead of Taq DNA polymerase used in common PCR which has a standard extension rate of 1 kb per minute, Fast PCR may be achieved using Taq DNA polymerase, which has a standard extension rate of 2-4 kb per minute.

In one embodiment, the amplification of a target nucleic acid may be performed by the Fast PCR method, and the Fast PCR method may include repeated cycles of up to 25 seconds or less, which includes a denaturation step, a primer annealing step, and an extension (or amplification) step.

In one embodiment, the denaturation step, the primer annealing step, and the extension step may be performed at the same or different temperatures. For example, the temperature at which the denaturation step is performed may be higher than the temperature at which the primer annealing step is performed and the temperature at which the extension step is performed. In particular, the temperature at which the primer annealing step is performed may be identical to the temperature at which the extension step is performed.

In one embodiment, the total number of cycles in the Fast PCR method may be, for example, 10 to 100, 10 to 90, 10 to 80, 10 to 70, 10 to 60, 10 to 50, 10 to 45, 20 to 100, 20 to 90, 20 to 80, 20 to 70, 20 to 60, 20 to 50, 20 to 45, 30 to 100, 30 to 90, 30 to 80, 30 to 70, 30 to 60, 30 to 50, or 30 to 45 cycles, and specifically, 40 to 45 cycles.

In one embodiment, the time required for each cycle may be 25 seconds or less, and specifically, 19 seconds, 18 seconds, 17 seconds, 16 seconds, 15 seconds, 14 seconds, 13 seconds, 12 seconds, 11 seconds, 10 seconds, 9 seconds, 8 seconds, 7 seconds, 6 seconds, or 5 seconds or less, but is not limited thereto.

In one embodiment, the time required for performing the denaturation step in each cycle may be 5 seconds or less, and specifically, 5 seconds, 4 seconds, 3 seconds, 2 seconds, or 1 second.

In one embodiment, the time required for performing the primer annealing step in each cycle may be 10 seconds or less, and specifically, 10 seconds, 9 seconds, 8 seconds, 7 seconds, 6 seconds, 5 seconds, 4 seconds, 3 seconds, 2 seconds, or 1 second.

In one embodiment, the time required for performing the extension step in each cycle may be 10 seconds or less, and specifically, 10 seconds, 9 seconds, 8 seconds, 7 seconds, 6 seconds, 5 seconds, 4 seconds, 3 seconds, 2 seconds, or 1 second.

In one embodiment, the combined time required for performing the primer annealing step and extension step in each cycle is 20 seconds or less, and specifically, 20 seconds, 18 seconds, 16 seconds, 14 seconds, 12 seconds, 10 seconds, 9 seconds, 8 seconds, 7 seconds, 6 seconds, 5 seconds, 4 seconds, 3 seconds, or 2 seconds.

In one embodiment, each cycle of the Fast PCR method may have a reaction temperature and/or reaction time different from one another.

In one embodiment, all of the cycles of the Fast PCR method may have a mutually-identical reaction temperature and/or reaction time.

In one embodiment, some of the cycles of the Fast PCR method may have a mutually-identical reaction temperature and/or reaction time.

In one embodiment, the step (c) is performed by repeating 40 to 45 cycles of the process of 2 to 5 seconds at 95°C and 5 to 20 seconds at 60°C.

In a certain embodiment, the step (c) is performed by repeating 5 cycles of the process of 5 seconds at 95°C and 20 seconds at 60°C, followed by repeating 40 cycles of the process of 2 seconds at 95°C and 5 seconds at 60°C.

In the case of a nucleic acid amplification reaction involving a temperature change, it is generally performed using a thermocycler. In the step (c) above, the total ramping time for each cycle, which includes the time required to reach the temperature for a denaturation step (i. e. a ramping time), the time required to reach the temperature for a primer annealing step, and the time required to reach the temperature for an extension step, is additionally required, and this depends on the ramping speed of the thermocycler.

In one embodiment, the total ramping time for each cycle may be 50 seconds or less. Specifically, the total ramping time for each cycle may be 45 seconds or less, 40 seconds or less, 35 seconds or less, 30 seconds or less, 25 seconds or less, 20 seconds or less, or 15 seconds or less.

In one embodiment, the reaction time of the step (c) is 6 to 47 minutes. The reaction time of the step (c) may be shortened or extended depending on the number of cycles, the time for denaturation, primer annealing, and extension within the cycle, the ramping time, *etc.*

Meanwhile, the total ramping time described above includes not only the ramping time within the cycle of the step (c), but further includes the ramping time between cycles of the steps (a) to (d).

In one embodiment, the steps (a-1), (b), and (c) are performed for a total of 12 to 60 minutes. The total reaction time may be shortened or extended depending on the number of cycles in the step (c), the total time for denaturation, primer annealing, and extension, and the total ramping time within the cycle of the step (c), *etc.*

In one embodiment, the method for detecting amplicons may be performed by a post-PCR detection method or a real-time detection method.

The post-PCR detection method is a method for detecting amplicons after the amplification of nucleic acids. The post-PCR detection method includes separation of the amplicons by size differences (generally performed using gel electrophoresis) or separation of amplicons by immobilization, but is not limited thereto.

Additionally, as the post-PCR detection method, a post-PCR melting assay (U.S. Patent Nos. 5,871,908 and 6,174,670 and WO 2012/096523), in which after amplifying the target nucleic acid sequence, the fluorescence intensity is monitored while increasing or decreasing the temperature during a certain period, and then amplicons are detected by a melting profile, may be used.

The real-time detection method can be performed using a non-specific fluorescent dye that is non-specifically intercalated into a duplex, which is an amplicon of the target nucleic acid sequence.

Additionally, in the real-time detection method, a labeled probe that specifically hybridizes with a target nucleic acid sequence may be used. For example, the above method includes a molecular beacon method using dual-labeled probes forming a hairpin structure (Tyagi et al., Nature Biotechnology v.14, March 1996), a hybridization probe method using two probes singly labeled as a donor or acceptor (Bernad et al., 147-148 Clin Chem 2000; 46), and the Lux method using a single labeled oligonucleotide (U.S. Patent No. 7,537,886), and the TaqMan method which utilizes not only the hybridization of a dual-labeled probe but also the cleavage reaction of the dual-labeled probe by the 5'-nuclease activity of DNA polymerase (U.S. Patent Nos. 5,210,015 and 5,538,848), but the method is not limited thereto.

Additionally, the real-time detection may be performed using a duplex generated depending on the presence of a target nucleic acid sequence. The duplex generated depending on the presence of a target nucleic acid sequence is not an amplicon of the target sequence itself generated through an amplification reaction, but is a duplexwhose amount increases in proportion to the amplification of the target nucleic acid sequence. The duplex generated depending on the presence of a target nucleic acid sequence may be obtained by various methods, for example, by the PTO Cleavage and Extension (PTOCE) method disclosed in WO 2012/096523, which is incorporated herein by reference.

Additionally, for the real-time detection of a target in the present disclosure, a method, in which one or more target nucleic acid sequences with only a single type of label are detected by using signal detection at different temperatures, may be used. This is disclosed in WO 2015/147412, which is incorporated herein by reference.

In one embodiment, the method may be performed by a reverse transcription PCR (RT-PCR) method, and more specifically, it may be performed by a one-step RT-PCR method.

In one embodiment, the method comprises a multiplex amplification reaction to amplify a plurality of target nucleic acids.

As used herein, the term "multiplex amplification reaction" refers to a reaction that targets and amplifies two or more nucleic acid sequences. That is, a multiplex amplification reaction refers to a reaction in which two or more nucleic acid sequences are amplified together in a single container by a single reaction. For example, the multiplex amplification reaction may be performed by the various nucleic acid amplification methods, post-PCR detection methods, and/or real-time detection methods described above.

In one embodiment, the method detects a plurality of target nucleic acids.

In another aspect of the present disclosure, there is provided a composition for detecting a target nucleic acid in a sample, which comprises:
(a) an oligonucleotide capable of hybridizing with the target nucleic acid;
(b) a thermostable Sso7d fusion DNA polymerase; and
(c) an antibody specifically bound to the thermostable Sso7d fusion DNA polymerase for inhibiting its activity.

The composition for detecting a target nucleic acid of an RNA virus of the present disclosure is intended to perform the method for detecting an RNA virus in the sample of the present disclosure described above, and the descriptions regarding common contents between the two are omitted herein in order to avoid excessive complexity of the present specification.

In one embodiment, the thermostable Sso7d fusion DNA polymerase comprises an epitope domain for the antibody.

In one embodiment, the antibody is denatured at 65°C or above.

In one embodiment, the denaturation of the antibody restores the activity of the thermostable Sso7d fusion DNA polymerase.

In one embodiment, the target nucleic acid is RNA.

In one embodiment, when the target nucleic acid is RNA, the composition further comprises a reverse transcriptase.

In one embodiment, the composition further comprises dNTPs and a buffer.

In one embodiment, the buffer comprises a Tris buffer of pH 7 to pH 8.

In one embodiment, the composition further comprises MgCl₂.

In one embodiment, the composition further comprises uracil DNA glycosylase (UDG).

In one embodiment, the composition further comprises an RNase inhibitor.

In one embodiment, the composition further comprises a stabilizer.

Unless otherwise defined in this specification, all technical and scientific terms used herein have the same meaning as commonly understood by those skilled in the art.

Hereinafter, the present disclosure will be described in more detail through examples. These examples are intended to explain the present disclosure in more detail, and it will be apparent to those skilled in the art to which the present disclosure pertains that the scope of the present disclosure proposed in the accompanying claims is not limited by these Examples.

### Examples

### Example 1. Preparation of samples

As target nucleic acids, the E gene, RdRP gene, and N gene of the RNA virus SARS-CoV-2 were used. For this purpose, plasmid DNA, which includes the sequences of each of the E gene, RdRP gene, and N gene of SARS-CoV-2 (wild-type) and the T7 promoter sequence, was synthesized from IDT. Then, the E gene template, RdRP gene template, and N gene template of SARS-CoV-2 were prepared by *in vitro* transcription using T7 RNA polymerase.

Thereafter, the prepared gene templates were each added to the SARS-CoV-2 negative sample stored at -80°C as shown in [Table 1], and thereby four kinds of SARS-CoV-2 positive samples (Sample Nos. 1 to 4 in Table 1) having a total of four concentrations (5 × 10³ copies/rxn, 5 × 10² copies/rxn, 5 × 10¹ copies/rxn, and 5 × 10⁰ copies/rxn) and a SARS-CoV-2 negative sample (Sample No. 5 in Table 1) were prepared. For the SARS-CoV-2 negative sample, a nasopharyngeal swab and an oropharyngeal (throat) swab were collected from normal person using a specimen collection kit and a universal transport medium (UTM, COPAN, 360C), pooled in one UTM (Universal Transport Medium, COPAN, 360C), and stored at -80°C.

**[Table 1]**

| Sample type +: Presence of template / -: Absence of template | | | | | | |
|---|---|---|---|---|---|---|
| Target Nucleic Acid | | Sample No. | | | | |
| | | 1 | 2 | 3 | 4 | 5 |
| SARS-CoV-2 | E gene | + | - | - | + | - |
| | RdRP gene | - | + | - | + | - |
| | N gene | - | - | + | + | - |

Then, an extraction kit (STARMag 96 × 4 Universal Cartridge Kit, Seegene) and 300 µL of each Sample No. 1 to Sample No. 5 of the four concentrations prepared above were mounted to an automated extraction equipment (Microlab NIMBUS IVD, Hamilton), and then nucleic acids were extracted with an elution volume of 100 µL.

### Example 2. Preparation of composition for detecting target nucleic acid of SARS-CoV-2

In order to detect the E gene, RdRP gene, and N gene of SARS-CoV-2, which are target nucleic acids, 5 µL of oligonucleotides capable of hybridizing with each of the target nucleic acids, 5 µL of an enzyme mix (including 8 U of- thermostable Sso7d fusion DNA polymerase bound to antibody, 7.5 U of an RTase, 0.1 U of UDG, and 400 mM Tris buffer at pH 7.6), and distilled water including 3.2 mM dNTPs, 30 mM MgCl₂, and 20 mM (NH₄)₂SO₄ were mixed, to prepare a composition for detecting the target nucleic acid of SARS-CoV-2.

As the oligonucleotides (including primers and probes) capable of hybridizing with the target nucleic acids of SARS-CoV-2, the oligonucleotides of Allplex^{™} SARS-CoV-2 Master Assay (Cat. No. RV10284X, Seegene Inc.), which is a multiple one-step RT-PCR product that can detect a plurality of targets in a single tube, were used.

The thermostable Sso7d fusion DNA polymerase, which is a Taq DNA polymerase having Sso7d fused to its N-terminus and a hot-start antibody specifically bound to the same, was prepared by the method described below.

First, the wild-type Taq DNA polymerase gene having wild-type Sso7d fused to its N-terminus was introduced into *E. coli* and expressed as a protein. The cells obtained by centrifugation were resuspended in Tris-HCl buffer (pH 8.0) and disrupted by ultrasonication. After centrifugation, the supernatant was heat-treated at 75 to 80°C. The obtained supernatant was loaded on a Ni-NTA affinity chromatography column, and the enzyme was eluted with Tris-HCl buffer (pH 8.0) including 0.15 M imidazole. The recovered fraction was dialyzed against a storage buffer including 20 mM Tris-HCl (pH 8.0), 100 mM KCl, 1 mM DTT, and 0.1 mM EDTA, and mixed with 0.5% Tween20, 0.5% NP-40, 1 mM PMSF, and 50% glycerol and stored. The purified thermostable Sso7d fusion DNA polymerase and a hot-start antibody (Cat. No. S132, GENEON) were mixed to prepare a thermostable Sso7d fusion DNA polymerase to which the hot-start antibody was bound.

The RTase used was the product of ThermoFisher (Cat. No. 18080044), and UDG used was the product of Enzynomics (Cat. No. M013).

### Example 3. One-step RT-PCR

15 µL of the composition for detecting the target nucleic acids of SARS-CoV-2 prepared in Example 2 and 5 µL of the nucleic acid extract sample prepared in Example 1 were mixed to prepare 20 µL of a final reaction mixture.

Tubes including each of the reaction mixtures prepared above were placed in a real-time thermocycler (CFX96, Bio-Rad) and allowed to react at 50°C for 10 minutes to perform a reverse transcription reaction, and then, a reaction was performed at 95°C for 3 minutes to induce denaturation of the antibody and restore the activity of thermostable Sso7d fusion DNA polymerase. Subsequently, 5 cycles of the process of 5 seconds at 95°C and 20 seconds at 60°C were repeated, and then, 40 cycles of the process of 2 seconds at 95°C and 5 seconds at 60°C were repeated to amplify the target nucleic acids. The detection of the signal was performed at 60°C for each cycle. The signal detected at the above temperature was analyzed using Seegene viewer software, which is a program for analysis. The above experiment was repeated twice for all samples to obtain the average Ct value (Tables 2 and 3).

As a result, as shown in [Table 2] and [Table 3], it was confirmed that the target nucleic acids of SARS-CoV-2 could be detected within 1 hour using the method according to the present disclosure. Additionally, it was confirmed that not only a single target nucleic acid (Sample No. 1 to Sample No. 3), but also a plurality of target nucleic acids (Sample No. 4) could be detected within 1 hour.

**[Table 2]**

| Average Ct value | | | | | | | | | |
|---|---|---|---|---|---|---|---|---|---|
| Gene Concentration (copies/rxn) | Sample No. 1 | | | Sample No. 2 | | | Sample No. 3 | | |
| | E gene | RdRP gene | N gene | E gene | RdRP gene | N gene | E gene | RdRP gene | N gene |
| 5 × 10³ | 25.41 | N/A | N/A | N/A | 26.71 | N/A | N/A | N/A | 27.84 |
| 5 × 10² | 28.2 | N/A | N/A | N/A | 30.43 | N/A | N/A | N/A | 31.145 |
| 5 × 10¹ | 31.98 | N/A | N/A | N/A | 34.04 | N/A | N/A | N/A | 35.13 |
| 5 × 10⁰ | 34.68 | N/A | N/A | N/A | 37.38 | N/A | N/A | N/A | N/A |

**[Table 3]**

| Average Ct value | | | | | | |
|---|---|---|---|---|---|---|
| Gene Concentration (copies/rxn) | Sample No. 4 | | | Sample No. 5 | | |
| | E gene | RdRP gene | N gene | E gene | RdRP gene | N gene |
| 5 × 10³ | 25.53 | 26.74 | 27.9 | N/A | N/A | N/A |
| 5 × 10² | 28.685 | 30.2 | 31.1 | N/A | N/A | N/A |
| 5 × 10¹ | 31.62 | 33.665 | 34.81 | N/A | N/A | N/A |
| 5 × 10⁰ | 36.315 | 37.845 | N/A | N/A | N/A | N/A |

## Claims

1. A method for detecting a target nucleic acid in a sample, comprising:
(a) preparing a reaction mixture by mixing the sample with a composition for detecting the target nucleic acid;
wherein the composition comprises: (i) an oligonucleotide capable of hybridizing with the target nucleic acid, (ii) a thermostable Sso7d fusion DNA polymerase and (iii) an antibody specifically bound to the thermostable Sso7d fusion DNA polymerase for inhibiting its activity,
(b) incubating the reaction mixture at 65°C or higher to induce denaturation of the antibody;
wherein the denaturation of the antibody restores the activity of the thermostable Sso7d fusion DNA polymerase; and
(c) amplifying the target nucleic acid in the reaction mixture.

2. The method of claim 1, wherein the sample is a swab sample, a spit sample, or a combination thereof.

3. The method of claim 2, wherein the swab sample is a nasopharyngeal swab sample, a nasal swab sample, an oropharyngeal swab sample, a saliva swab sample or a combination thereof.

4. The method of claim 1, wherein the sample is subjected to a crude extraction process prior to the step (a).

5. The method of claim 4, wherein the crude extract process comprises incubating the sample at 95°C to 100°C for 1 to 25 minutes.

6. The method of claim 1, wherein the thermostable Sso7d fusion DNA polymerase comprises an epitope domain for the antibody.

7. The method of claim 1, wherein the target nucleic acid is RNA.

8. The method of claim 7, wherein the method further comprises step (a-1) of incubating the reaction mixture at 45°C to 55°C before the step (b) to reverse transcribe the target nucleic acid.

9. The method of claim 7, wherein the composition further comprises a reverse transcriptase.

10. The method of claim 1, wherein the composition further comprises dNTPs and a buffer.

11. The method of claim 10, wherein the buffer comprises a Tris buffer in the pH range of 7.0-8.0.

12. The method of claim 11, wherein the Tris buffer is comprised in the reaction mixture at a concentration of 60 mM to 120 mM.

13. The method of claim 1, wherein the composition further comprises MgCl₂.

14. The method of claim 13, wherein MgCl₂ is comprised in the reaction mixture at a concentration of 6 mM to 9 mM.

15. The method of claim 13, wherein MgCl₂ is separated from the thermostable Sso7d fusion DNA polymerase prior to the step (a).

16. The method of claim 9, wherein the composition further comprises MgCl₂, and MgCl₂ is separated from the reverse transcriptase prior to the step (a).

17. The method of claim 1, wherein the composition further comprises uracil DNA glycosylase (UDG).

18. The method of claim 1, wherein the composition further comprises an RNase inhibitor.

19. The method of claim 1, wherein the composition further comprises a stabilizer.

20. The method of claim 8, wherein the step (a-1) is performed for 5 to 10 minutes.

21. The method of claim 1, wherein the step (b) is performed for 1 to 3 minutes.

22. The method of claim 1, wherein the step (c) is performed for 6 to 47 minutes.

23. The method of claim 1, wherein the step (c) is performed for 40 to 45 cycles of the process of 2 to 5 seconds at 9°C and 5 to 20 seconds at 60°C.

24. The method of claim 8, wherein the steps (a-1), (b) and (c) are performed for a total of 12 to 60 minutes.

25. The method of claim 1, wherein the method detects a plurality of target nucleic acids.

26. A composition for detecting a target nucleic acid in a sample, comprising:
(a) an oligonucleotide capable of hybridizing with the target nucleic acid;
(b) a thermostable Sso7d fusion DNA polymerase; and
(c) an antibody specifically bound to the thermostable Sso7d fusion DNA polymerase for inhibiting its activity.

27. The composition of claim 26, wherein the thermostable Sso7d fusion DNA polymerase comprises an epitope domain for the antibody.

28. The composition of claim 26, the antibody is denatured at 65°C or higher.

29. The composition of claim 28, wherein the denaturation of the antibody restores the activity of the thermostable Sso7d fusion DNA polymerase.

30. The composition of claim 26, wherein the target nucleic acid is RNA.

31. The composition of claim 30, wherein the composition further comprises a reverse transcriptase.

32. The composition of claim 26, wherein the composition further comprises dNTPs and a buffer.

33. The composition of claim 32, wherein the buffer comprises a Tris buffer in the pH range of 7.0-8.0.

34. The composition of claim 26, wherein the composition further comprises MgCl₂.

35. The composition of claim 26, wherein the composition further comprises uracil DNA glycosylase (UDG).

36. The composition of claim 26, wherein the composition further comprises an RNase inhibitor.

37. The composition of claim 26, wherein the composition further comprises a stabilizer.
